**Europäisches Patentamt**

**European Patent Office**

**Office européen des brevets**

(19)

(11) Veröffentlichungsnummer: **0 262 379**
**B1**

(12) # EUROPÄISCHE PATENTSCHRIFT

(45) Veröffentlichungstag der Patentschrift: **23.01.91**

(51) Int. Cl.⁵: **A 61 F 2/34**

(21) Anmeldenummer: **87112218.0**

(22) Anmeldetag: **22.08.87**

(54) Endoprothese für eine Hüftgelenkspfanne.

(30) Priorität: **23.09.86 CH 3799/86**

(43) Veröffentlichungstag der Anmeldung:
**06.04.88 Patentblatt 88/14**

(45) Bekanntmachung des Hinweises auf die
Patenterteilung:
**23.01.91 Patentblatt 91/04**

(84) Benannte Vertragsstaaten:
**AT DE FR GB IT**

(56) Entgegenhaltungen:
**EP-A-0 065 482**
**WO-A-86/02261**
**DE-C-2 411 617**
**DE-C-3 341 723**
**FR-A-2 548 012**

(73) Patentinhaber: **GEBRÜDER SULZER
AKTIENGESELLSCHAFT
Zürcherstrasse 9
CH-8401 Winterthur (CH)**

(72) Erfinder: **Imhof Martin
Schöngrund 10
CH-6343 Rotkreuz (CH)**
Erfinder: **Willert, Hans-Georg, Prof. Dr.-med.
Schlegelweg 9
D-3400 Göttingen (DE)**

(74) Vertreter: **Sparing Röhl Henseler Patentanwälte
European Patent Attorneys
Rethelstrasse 123
D-4000 Düsseldorf 1 (DE)**

EP 0 262 379 B1

# Beschreibung

Die Erfindung betrifft eine Endoprothese für eine Hüftgelenkspfanne, mit einem Pfannenkörper, der eine eine Äquatorebene aufweisende Pfannenschale ausbildet, auf seinem Außenmantel ein selbschneidendes Schraubengewinde trägt und im Polbereich im wesentlichen eine Ebene senkrecht zur Pfannenachse bildet.

Die Außenform vom im Bechenknochen zu implantierenden künstlichen Hüftgelenkspfannen der vorstehend genannten Art ist bisher entweder als Kegelstumpf oder als in dem Polbereich "gekappte" Halbkugel ausgebildet. Als Beispiel aus der Vielzahl der bekannten Konstruktionen seien für die Kegelstumpfform die DE—C—24 11 617 und für die Halbkugel die EP—A—00 65 482 genannt.

Beim Einsetzen, Einschlagen oder Einschrauben der Pfannen in den Beckenknochen übt der Außenmantel unter anderem eine Druckkraft auf den Knochen aus, deren Wirkungslinie senkrecht zu seiner Oberfläche ist. Zerlegt man diese Kraft C in eine Komponente a senkrecht und eine Komponente b parallel zur Pfannenachse, so ist das Verhältnis dieser Komponenten bei einer Kegelstumpfform konstant, während es sich bei der Kugelpfanne zwischen äquatorialen und polnahen "Breiten" sehr rasch in dem Sinne ändert, daß vom Äquator zum Pol die Komponente senkrecht zur Pfannenachse relativ schnell ab- und die Parallelkomponente zunimmt.

Beide Pfannenformen verhalten sich daher beim Implantieren unterschiedlich: Die Kegelstumpfform besitzt eine hohe Stabilität gegen ein Verkanten und Abkippen beim Einsetzen in eine an ihre Form angepaßte, operativ vorbereitete Ausnehmung im Beckenknochen. Dafür muß in Kauf genommen werden, daß keine Korrekturmöglichkeit für die Richtung der Pfannenachse mehr besteht, wenn die Ausnehmung "gesetzt" ist. Weiterhin ergibt sich bei einem zu starken Anziehen die Gefahr, daß das Becken gesprengt wird. Bei der Kugelform ist in einem gewissen Umfang eine Korrektur der Achsrichtung möglich, was jedoch gleichzeitig eine Instabilität gegen ein unbeabsichtigtes Verkanten und Abkippen einschließt. Darüberhinaus reißt beim Einschrauben, bei dem ein Gewinde in den Knochen geschnitten wird, das Gewinde aus, bevor es zu einer Sprengung des Beckens kommt.

Der Erfindung liegt die Aufgabe zugrunde, eine Pfanne der genannten Art zu schaffen, die die Vorteile der beiden geschilderten Formen vereinigt, die Nachteile jedoch vermeidet.

Diese Aufgabe wird dadurch gelöst, daß die äußere Oberfläche des Pfannenkörpers und/oder die Umhüllende des Schraubengewindes im Querschnitt von der Äquatorebene bis zum Mittelpunkt der polseitigen Ebene den Ausschnitt eines Hyperbelastes bilden, dessen Symmetrieachse durch den Scheitelpunkt des Hyperbelastes einen Winkel zwischen 48° und 58° mit der Äquatorebene bildet, so daß ein Schenkel des Hyperbelastes in der polseitigen Ebene verläuft, während der andere die Mantellinie des Pfannenkörpers bzw. der Umhüllenden für das Gewinde ist.

Im polnahen Bereich ergibt sich so ein relativ großes Verhältnis a/b. Daher wird auch bei der erfindungsgemäßen Pfanne in diesem Bereich das Gewinde zerstört, ehe es zur Sprengung des Beckens kommt. Zum Äquator hin nähert sich die hyperbolische Form einer Kegelstumpfform. Im äquatornahen Teil des Außenmantels wird daher eine Stabilität gegen ein Abkippen erreicht. Die Möglichkeit für eine Korrektur der Richtung der Pfannenachse während der Operation ist in gewissem Umfang gegeben.

Für den Einsatz eines Einschraubinstrumentes kann der Pfannenkörper vier auf seinem äquatorialen Rand verteilte Nuten haben, die unter einem Winkel zu zur Pfannenachse parallelen Mantellinien verlaufen. Durch die Schräge der Nut wird eine sichere Fixierung der Prothese am Einschraubinstrument gewährleistet.

Bei Konstruktionen, die aus einer metallenen Außenschale und einem inneren, die eigentliche Pfannenschale enthaltenden Schalenkörper aus Kunststoff bestehen, wobei die Gesamtpfanne werkstattmontiert sterilisiert geliefert wird—d.h. das Einsetzen des Schalenkörpers nicht erst intraoperativ erfolgt, —ist es zweckmäßig, wenn im stirnseitigen äquatorialen Rand des Schalenkörpers Sackbohrungen vorgesehen sind, die für den Eingriff des Einschraubinstruments auf die Nuten und ihre Schräge abgestimmt sind.

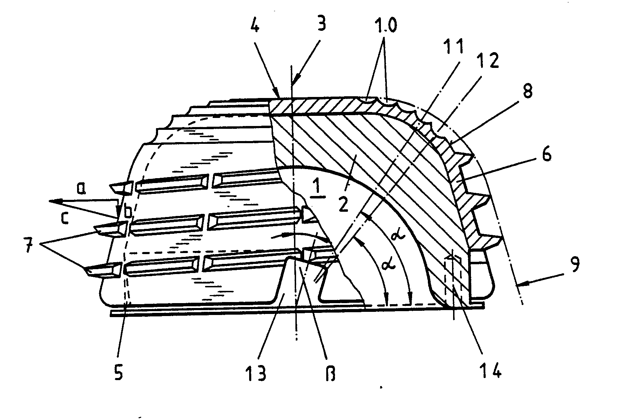

Im folgenden wird die Erfindung anhand eines Ausführungsbeispiels im Zusammenhang mit der beigefügten Zeichnung erläutert, die eine Endoprothese für eine Hüftgelenkspfanne teilweise im Schnitt zeigt.

Die einen nicht gezeigten Gelenkkopf aufnehmende eigentliche Pfannenschale 1 befindet sich in einem inneren Schalenkörper 2 aus Kunststoff, z.B. Polyäthylen. Ihre Achse 3 fällt mit der Rotationssymmetrie- oder Pfannenachse zusammen.

Der Schalenkörper 2 ist in eine metallene Hülse 4, beispielsweise aus Titan oder einer Titanlegierung, eingepreßt oder eingeschrumpft, wobei ein "negativer" d.h. ein sich in richtung zum Pol der Pfannenschale 1 hin erweiternder Konus 5 am Schalenkörper 2 und an der Hülle 4 einen zusätzlichen Halt ergibt.

Der Außenmantel 6 der Hülle 4 ist mit einem selbstschneidenden Gewinde 7 versehen, das mehr- oder—wie gezeigt—eingängig ausgebildet sein kann, und bildet im Polbereich eine Ebene senkrecht zur Pfannenachse 3 gegenüber von der Äquatorebene der Pfanne.

Erfindungsgemäß bildet sowohl die äußere Oberfläche 8 des Außenmantels 6 als auch die Umhüllung 9 für das Gewinde 7 im Querschnitt von der Äquatorebene bis zum Mittelpunkt der polseitigen Ebene den Ausschnitt jeweils eines Hyperbelastes, von dem ein Schenkel in der sich senkrecht zur Pfannenachse 3 erstreckenden polseitigen Ebene verläuft. Letztere ist teilweise durch zur Achse 3 konzentrische Rillen 10 strukturiert, die das Anwachsen von Gewebe fördern. Die Symmetrieachsen 11 bzw. 12 des jeweiligen

2

Hyperbelastes bilden mit der Äquatorebene im vorliegenden Beispiel einen Winkel α von etwa 55° bzw. 51°.

Im Rand der Hülle 4 sind auf dem Umfang gleichmäßig vier Nuten 13 verteilt, deren Längsachsen mit parallel zur Pfannenachse verlaufenden Mantellinien der Hülle 4 einen Winkel β bilden. Die Nuten 13 dienen zum Eingriff eines nicht gezeigten Einschraubinstrumentes, wobei die Schrägstellung ihrer Längsachsen eine sichere Fixierung zwischen beiden gewährleistet.

Bei der gezeigten Konstruktion aus einem Schalenkörper 2 und einer Hülle 4 ist es weiterhin notwendig, beim Einschrauben der Pfanne in den Knochen eine Relativbewegung zwischen Schalenkörper 2 und Hülle 4 zu verhindern. Im stirnseitigen Rand des Schalenkörpers 2 sind daher Sackbohrungen 14 vorgesehen, die auf die Nuten 13 und ihre Schräge derart abgestimmt sind, daß ein an der Hülle 4 fixiertes Einschraubinstrument zusätzlich in die Bohrung 14 eingreift.

Selbstverständlich ist die Erfindung nicht an die gezeigte und beschriebene zweiteilige Ausführung einer Prothese für eine Hüftgelenkspfanne gebunden. Sie Kann in gleicher Weise auch bei Konstruktionen angewandt werden, bei denen die Prothese aus einem Pfannenkörper, z.B. aus Metall, besteht, der sowohl die Pfannenschale als auch die Außenstruktur enthält.

**Patentansprüche**

1. Endoprothese für eine Hüftgelenkspfanne mit einem Pfannenkörper, der eine eine Äquatorebene aufweisende Pfannenschale (1) ausbildet, auf seinem Außenmantel ein selbstschneidendes Schraubengewinde (7) trägt und im Polbereich im wesentlichen eine Ebene senkrecht zur Pfannenachse (3) bildet, dadurch gekennzeichnet, daß die äußere Oberfläche (8) des Pfannenkörpers und/oder die Umhüllende (9) des Schraubengewindes (7) im Querschnitt von der Äquatorebene bis zum Mittelpunkt der polseitigen Ebene den Ausschnitt eines Hyperbelastes bildet, dessen Symmetrieachse (11, 12) durch den Scheitelpunkt des Hyperbelastes einen Winkel (α) zwischen 48° und 58° mit der Äquatorebene bildet, so daß ein Schenkel des Hyperbelastes in der polseitigen Ebene verläuft, während der andere die Mantellinie des Pfannenkörpers bzw. der Umhüllenden (9) für das Schraubengewinde (7) ist.

2. Endoprothese nach Anspruch 1, dadurch gekennzeichnet, daß in den stirnseitigen, die Pfannenschale (1) umgebenden Rand des Pfannenkörpers gleichmäßig auf dem Umfang verteilte Nuten (13) eingearbeitet sind, die gegenüber zur Pfannenachse (3) parallelen Mantellinien des Außenmantels (6) geneigt sind.

3. Endoprothese nach Anspruch 2, dadurch gekennzeichnet, daß der Pfannenkörper aus einer metallenen Hülle (4) und einem inneren Schalenkörper (2) aus Kunststoff besteht, die werkstattmontiert miteinander verbunden sind, und daß in der Stirnseite des Schalenkörpers (2) auf die Nuten (13) und ihre Schräge abgestimmte Sackbohrungen (14) vorgesehen sind.

**Revendications**

1. Endoprothèse pour cavité cotyloïde, comportant un corps de cavité qui forme une coque de cavité (1), présentant un plan d'équateur, qui porte, sur son enveloppe extérieure, une partie filetée (7) autotaraudeuse et qui forme en gros, dans la région polaire, un plan perpendiculaire à l'axe de cavité (3), caractérisée en ce que la surface extérieure (8) du corps de cavité et/ou l'enveloppe (9) de la partie filetée (7) forment, en coupe transversale, depuis le plan équatorial jusqu'au point central du plan polaire, la découpe d'une hyperbole dont l'axe de symétrie (11, 12) passant par le sommet de l'hyperbole forme un angle α compris entre 48° et 58° avec le plan équatorial, de sorte qu'une branche de l'hyperbole s'étend dans le plan polaire, tandis que l'autre est la génératrice du corps de cavité ou de l'enveloppe (9) de la partie filetée (7).

2. Endoprothèse selon la revendication 1, caractérisée en ce que des rainures (13), qui sont inclinées par rapport à des génératrices de l'enveloppe extérieure (6), parallèles à l'axe de cavité (3), sont pratiquées dans le bord frontal du corps de cavité, entourant la coque de cavité (1), régulièrement réparties sur le pourtour.

3. Endoprothèse selon la revendication 2, caractérisée en ce que le corps de cavité est constitué par une gaine (4) métallique et par un corps de coque (2) intérieur en matière plastique qui sont assemblés entre eux en usine, et en ce qu'on prévoit des trous borgnes (14), adaptés aux rainures (13) et à leur inclinaison, dans la face frontale du corps de coque (2).

**Claims**

1. An acetabulum endoprothesis comprising a prosthetic socket or cup forming a shell (1) having an equatorial plane, the cup having a self-tapping screwthread (7) on its outside generated surface and forming in its pole region substantially a plane perpendicular to the acetabulum axes (3), characterised in that the outside surface (8) of the cup and/or the envelope (9) of the screwthread (7) forms in cross-section from the equatorial plane as far as the centre of the plane near the pole a part of a hyperbola branch whose axis (11, 12) of symmetry extending through the apex of such branch forms an angle between 48° and 58° with the equatorial plane so that one arm of such branch extends in the plane near the pole while the other arm is the generatrix of the cup or of the envelope (9) for the screwthread (7).

2. An endoprosthesis according to claim 1, characterised in that the end-face cup edge extending around the shell (1) is formed with peripherally distributed grooves (13) disposed at an inclination to generatrices of the outer generated surface (6) which are parallel to the acetabulum axis (3).

3. An endoprosthesis according to claim 2, characterised in that the cup is embodied by a metal sleeve or the like (4) and an inner plastics shell member (2) which are interconnected during workshop assembly, and the end face of the shell member (2) is formed with blind bores (14) adapted to the grooves (13) and to their inclination.